# DEMANDE DE BREVET EUROPEEN

(11) **EP 0 564 373 A1**
(43) Date de publication de la demande: **06.10.1993**
(21) Numéro de dépôt: 93400870.7
(22) Date de dépôt: 02.04.1993
(51) Int. Cl.: A61B 17/34, A61B 1/00

(54) **Trocart autobloquant**

(30) Priorité: 03.04.1992 FR 9204156
(71) Demandeur: Melin, Laurent, F-52800 Nogent en Bassigny (FR)
(72) Inventeur: Melin, Laurent, F-52800 Nogent en Bassigny (FR); Melin, Cyriaque, F-52800 Nogent en Bassigny (FR)
(74) Mandataire: Bruder, Michel

(57) **Abrégé**

La présente invention concerne un trocart notamment destiné à l'introduction et au maintien d'une instrumentation opératoire, du type de pinces (13) ou de systèmes optiques d'éclairage et d'exploration visuelle, dans un pneumopéritoine d'un patient opéré par coelioscopie, ce trocart comportant au moins un tube (1) destiné à être introduit au travers de la peau et du péritoine dudit patient pour procurer un passage coaxial à ladite instrumentation, caractérisé en ce que ledit tube (1) d'introduction est prolongé à sa partie proximale par un logement (2, 6) dans lequel est disposée une bague annulaire (3), normalement fermée par une membrane élastique (4) faisant étanchéité entre l'extérieur et le pneumopéritoine du patient, ladite membrane élastique (4) étant incisée de manière à pouvoir faire passer ladite instrumentation coaxialement au travers dudit tube (1), les lèvres de la membrane (4), ainsi formées par incision, procurant un serrage positif de la paroi cylindrique extérieure de ladite instrumentation d'une manière permettant, d'une part, d'assurer l'étanchéité et, d'autre part, d'interdire les mouvements relatifs longitudinaux dudit tube (1) et de ladite instrumentation dus à la simple gravité.

## Description

La présente invention concerne un trocart autobloquant notamment applicable à la coelioscopie.

On sait que la coelioscopie est une méthode d'exploration de la cavité péritonéale, préalablement insufflée d'un gaz stérile. Après une anesthésie générale ou une anesthésie locale de la paroi abdominale au voisinage de la région à examiner, on crée donc un pneumopéritoine ; dans cette poche gazeuse artificiellement maintenue sous pression par un insufflateur spécifique, on introduit un trocart spécial dont on retire le mandrin pour le remplacer par un système optique lumineux, ce qui permet d'explorer visuellement le foie, la vésicule biliaire, le péritoine, etc... Dans certains cas, d'autres trocarts que celui de l'endoscope sont introduits dans le pneumopéritoine, ce qui permet au praticien de procéder à des ponctions ou à des biopsies d'organes qu'il effectue à l'aide de pinces coelioscopiques longilignes qui sont introduites coaxialement au travers desdits trocarts.

On comprend que ce type d'intrumentation nécessite un double contrôle, d'une part, de l'étanchéité entre l'extérieur et le pneumopéritoine maintenu en pression et, d'autre part, du guidage et du maintien des pinces coelioscopiques et de l'endoscope au milieu de leurs trocarts respectifs. En effet, l'intérêt porté à la coelioscopie par les praticiens se développe et, devant la multiplication des instrumentations qu'ils utilisent, il est indispensable que les broches ou les pinces coelioscopiques puissent être fermement bloquées dans n'importe quelle position le long des trocarts, notamment à la verticale. En outre, les moyens de blocage doivent être suffisamment légers pour ne pas trop alourdir les instrumentations. Enfin, il est évident que les trocarts doivent pouvoir être facilement démontés pour leur stérilisation.

A ce jour, on ne connaît pas de trocarts répondant complétement à toutes ces exigences ; en particulier, les moyens de blocage actuellement disponibles sont très complexes, comportent un nombre excessif de pièces mécaniques, dont des ressorts, et présentent un poids important qui nuit à la stabilité des instruments.

La présente invention vise à remédier à ces inconvénients en proposant un nouveau trocart notamment destiné à l'introduction et au maintien d'une instrumentation opératoire, du type de pinces ou de systèmes optiques d'éclairage et d'exploration visuelle, dans un pneumopéritoine d'un patient opéré par coelioscopie, ce trocart comportant classiquement au moins un tube destiné à être introduit au travers de la peau et du péritoine dudit patient pour procurer un passage coaxial à ladite instrumentation, ce trocart étant caractérisé en ce que ledit tube est prolongé à sa partie proximale par un logement dans lequel est disposée une bague annulaire, normalement fermée par une membrane élastique faisant étanchéité entre l'extérieur et le pneumopéritoine du patient, ladite membrane élastique étant incisée de manière à pouvoir faire passer ladite instrumentation coaxialement au travers dudit tube, les lèvres de la membrane ainsi formées par incision procurant un serrage positif du manche de ladite instrumentation d'une manière permettant, d'une part, d'assurer l'étanchéité et, d'autre part, d'interdire les mouvements relatifs longitudinaux dudit tube et de ladite instrumentation dus à la simple gravité.

Dans une variante préférentielle de l'invention, la membrane élastique de la bague annulaire disposée dans le logement proximal du tube d'introduction du trocart présente une forme conique, évasée à sa partie distale, et fendue par son sommet en deux parties égales de manière à former une sorte de "bec de canard". Cette disposition de l'invention facilite l'introduction des instruments au travers de la membrane qui, si elle était plane, offrirait une résistance beaucoup plus importante à la déformation. En outre, on observera que, même s'il est tout à fait envisageable de prévoir une découpe de la membrane en plusieurs secteurs (trois, quatre ou plus), identiques ou non, il est préférable de fendre ladite membrane en seulement deux lèvres venant en appui positif l'une contre l'autre ; en effet, cet agencement permet d'éviter que, lors du retrait d'une instrumentation hors du tube d'introduction du trocart, le manche de cette dernière ne provoque un retournement au moins partiel des découpes en secteur de ladite membrane, créant un freinage ou un blocage indésirable lors du mouvement de retrait. En outre, on comprend qu'il est essentiel que l'étanchéité puisse être recouvrée immédiatemment après que l'on ait retiré l'instrumentation, ce qui nécessite que les lèvres de l'incision pratiquée dans la membrane retrouvent rapidement leur forme initiale et reviennent parfaitement au contact les unes des autres.

Afin d'accentuer encore l'effet de resserrement des deux parties du "bec de canard" l'une contre l'autre, et suivant une caractéristique complémentaire de l'invention, on donne une forme légèrement ovale ou oblongue à la paroi extérieure de la bague annulaire portant la membrane élastique fendue, avec un petit axe parallèle à l'incision pratiquée dans cette dernière ; étant donné que le logement proximal du tube d'introduction du trocart a normalement la forme d'un cylindre de révolution, la bague annulaire se retrouve pincée suivant son grand axe. De cette manière, les deux lèvres de l'incision sont sollicitées en appui positif l'une contre l'autre, ou contre le manche de l'instrumentation opératoire montée à l'intérieur du tube d'introduction du trocart, ce qui améliore subsidiairement l'étanchéité de la membrane élastique en forme de bec de canard.

Suivant une caractéristique importante de l'invention, la bague annulaire et sa membrane sont réalisées en un matériau élastomère incompressible, normalement utilisé comme ressort industriel. Ce matériau présente une dureté et un état de surface suffisants pour servir de frein lorsqu'il vient en appui élastique contre le manche des instruments, tout en ayant une élasticité adéquate pour reprendre sa forme initiale après déformation.

En outre, du fait de l'incompressibilité de cet élastomère, il est prévu de réaliser une échancrure circonférentielle située à la racine de la membrane élastique conique, cette échancrure facilitant le dégagement des deux parties du "bec de canard" vers l'extérieur lorsqu'un instrument traverse ladite membrane.

D'un autre côté, on comprend que l'extrémité distale des instruments qui sont introduits dans le pneumopéritoine sont particulièrement fragiles, ce qui est le cas des lentilles des endoscopes ou des mors des pinces coelioscopiques. C'est pourquoi, suivant une caractéristique avantageuse de la présente invention, le logement proximal du tube d'introduction du trocart est constitué par une douille cylindrique sur laquelle on vient visser un manchon fileté qui est prolongé, à sa partie proximale, par un tube de guidage servant à faire coulisser un tube intérieur d'ouverture et de fermeture du "bec de canard". Ce tube intérieur a pour rôle de provoquer l'ouverture de la membrane élastique juste avant le passage d'un instrument ; de cette manière, lorsque le trocart est en place dans le pneumopéritoine du patient, on peut enfoncer ledit instrument sans dommage au travers dudit trocart ; dans un second temps, on recule le tube intérieur, faisant en sorte de relâcher légèrement les lèvres de la membrane ouverte contre le manche de l'instrument ainsi mis en place. Ce dernier est alors fermement maintenu le long du trocart. L'opération inverse permet de retirer l'instrument également sans craindre de l'endommager.

De manière auxiliaire, on observera que le tube intérieur a pour rôle d'adapter le diamètre intérieur du tube d'introduction du trocart avec le diamètre extérieur du manche de l'instrumentation opératoire.

D'autres caractéristiques et avantages de la présente invention ressortiront mieux de la description qui va suivre d'un mode de réalisation préférentiel d'un trocart de coelioscopie donné à titre d'exemple non limitatif en référence aux dessins annexés sur lesquels :
- la figure 1 est une vue en coupe longitudinale du trocart en situation de montage,
- la figure 2 est une vue latérale extérieure, partiellement éclatée, du trocart monté, avec la membrane élastique en bec de canard en position de fermeture, un mandrin ayant été introduit coaxialement dans le tube intérieur,
- la figure 3 est une vue latérale extérieure, partiellement éclatée, du trocart monté, avec le bec de canard en position d'ouverture provoquée par le tube intérieur,
- la figure 4 est une vue de face du bec de canard ouvert, poussé par le tube intérieur du trocart,
- la figure 5 est une vue latérale extérieure, partiellement éclatée, du trocart, avec le bec de canard en position d'ouverture, les lèvres de la membrane élastique reposant sur la paroi extérieur du mandrin suite au retrait vers l'arrière du tube intérieur.

Le trocart pour la coelioscopie représenté sur les figures annexées est constitué d'un ensemble de cinq pièces, à savoir :
- un tube d'introduction 1 comportant, à sa partie proximale, une douille cylindrique 2, filetée extérieurement, d'un diamètre normalement supérieur à celui du tube d'introduction 1.
- une bague annulaire 3, de forme sensiblement cylindrique, destinée à s'engager dans la douille 2 ; cette bague annulaire 3 est fermée par une membrane élastique 4 en forme de cône, dont le sommet 5 est dirigé vers le tube d'introduction 1, et qui est fendue en deux parties identiques de manière à procurer un passage coaxial aux instruments chirurgicaux ou d'exploration de l'abdomen ; la membrane élastique 4 forme ainsi une sorte de "bec de canard" à l'intérieur de la bague annulaire 3.
- une pièce intermédiaire comportant un manchon cylindrique 6, fileté intérieurement pour venir se visser autour de la douille 2, en enserrant la bague annulaire 3, ledit manchon 6 étant prolongé, à sa partie proximale, par un tube de guidage 7 terminé par une collerette 8, en saillie extérieure par rapport audit tube 7.
- un tube intérieur 9 monté coulissant à l'intérieur du tube de guidage 7, et se terminant par une collerette 10, en saillie extérieure par rapport audit tube 9 ; la longueur de ce tube intérieur 9 est calculée de manière à ce que le bord libre distal dudit tube intérieur 9 vienne provoquer l'ouverture complète de la membrane élastique 4 lorsque les deux collerettes 8 et 10 sont amenées en butée l'une contre l'autre.
- un manchon annulaire élastique 11 comportant, à l'arrière, une couronne 12 de rayon intérieur adéquat pour former un joint torique servant à centrer et à freiner ou bloquer, par frottement, l'instrumentation, par exemple une pince coelioscopique 13, pouvant être introduite dans le tube intérieur 9.

Suivant l'invention, la bague annulaire 3 et sa membrane élastique 4 en bec de canard sont réalisées dans un matériau élastomère tel que, par exemple, celui commercialisé sous les marques Eladur et Eladip. Selon le résultat d'essais, l'épaisseur optimale de la membrane élastique 4 est de 1,4 millimètre. La bague annulaire 3 peut être moulée, ou encore usinée, suivant des procédés garantissant un état de surface le moins lisse possible. Préférentiellement, la dureté de l'élastomère choisi sera comprise entre 45 et 60 Shore.

Etant donné que ce type d'élastomère est parfaitement incompressible, on comprend que la souplesse de la membrane élastique 4 en bec de canard pourrait être gênée du fait que les deux parties symétriques de cette membrane 4 ne trouvent pas un dégagement suffisant, en position écartée, au milieu de la bague annulaire 3. De ce fait, on réalise une échancrure circonférentielle 14 à la racine de la membrane élastique conique 4, cette échancrure 14 facilitant le dégagement des deux parties du "bec de canard" vers l'extérieur.

Le manchon annulaire 11 pourra être réalisé dans le même matériau, toujours par usinage ou par moulage.

Suivant une caractéristique avantageuse de l'invention, le tube intérieur 9 présente une possibilité de déplacement coaxial limité à l'intérieur du tube de guidage 7. A cet effet, les collerettes 8, 10 respectives de ces deux tubes 7, 9 sont introduites dans un logement cylindrique 15, aménagé à l'intérieur du manchon annulaire élastique 11, ledit logement 15 étant délimité, à l'avant, par un bourrelet 16 venant serrer, d'une manière élastique, la paroi externe du tube de guidage 7. Ce bourrelet 16 sert également de butée pour la collerette terminale 8 dudit tube de guidage 7, lorsque le tube intérieur 9 est coulissé vers l'arrière du trocart. On comprend en effet que la course longitudinale du tube intérieur 9 doive être aussi limitée que possible pour que le trocart soit d'une utilisation aisée.

Enfin, on observera que le logement cylindrique intérieur 15 du manchon annulaire 11 comporte deux chambres délimitées par un jonc 17 venant faire étanchéité lorsque les collerettes 10, 8 respectives du tube intérieur 9 et du tube de guidage 7 viennent en butée l'une contre l'autre.

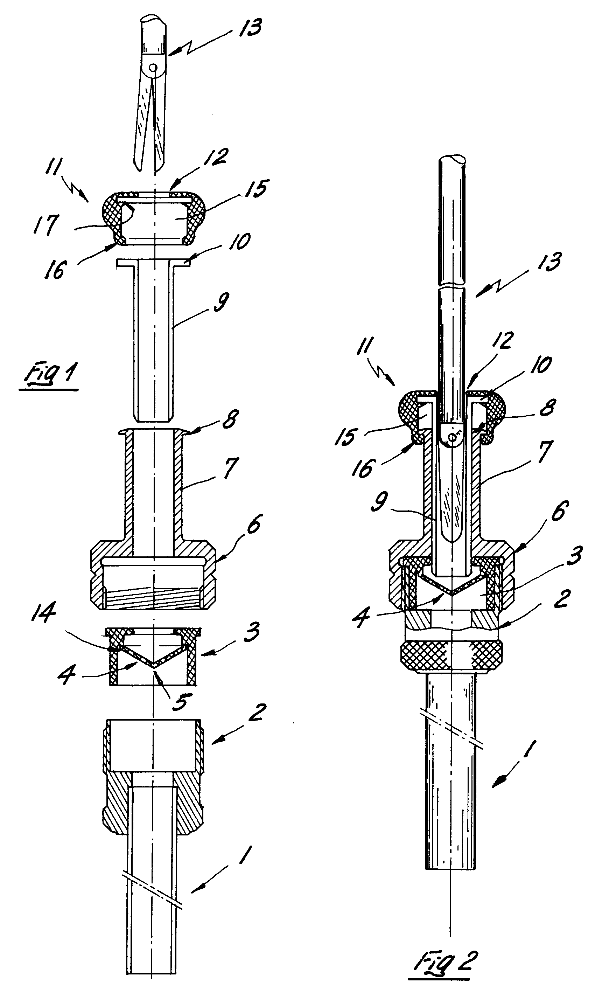

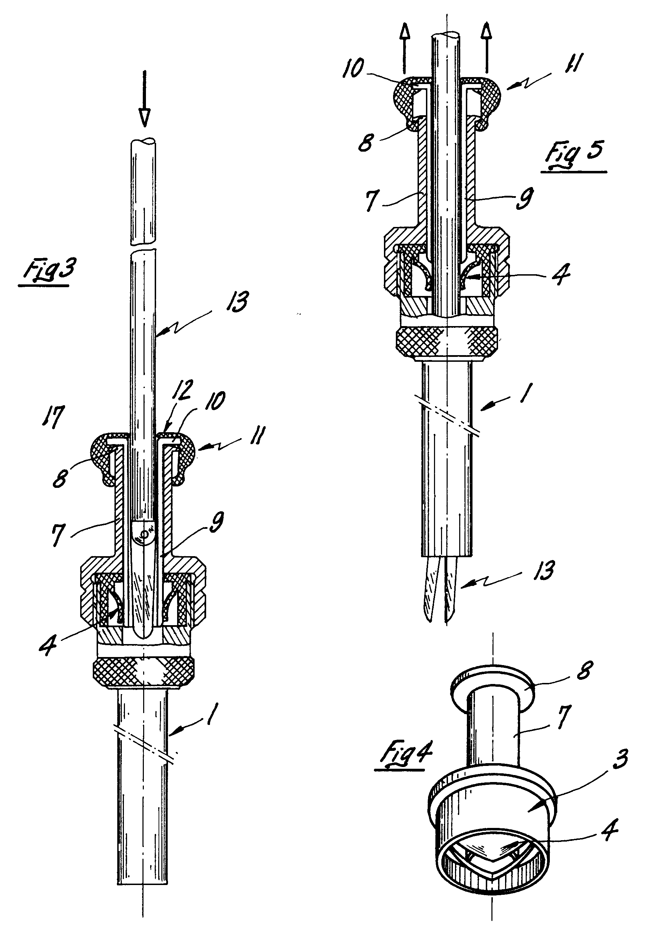

On expliquera maintenant plus en détail le fonctionnement du trocart conforme à l'invention, ceci en référence aux figures 2 à 5.

Une fois introduit dans le pneumopéritoine formé par insufflation d'un gaz stérile dans une partie de l'abdomen du patient, le trocart doit assurer l'étanchéité entre l'air extérieur et ledit pneumopéritoine. A cet effet, la membrane élastique 4 en bec de canard doit se trouver en position de fermeture, ce qui illustré sur la figure 2 où l'on a également représenté la pince coelioscopique 13 prête à être introduite au travers de ladite membrane 4 ; dans cette position initiale, le tube intérieur 9 se trouve le plus en arrière possible du tube de guidage 7, lequel est alors en appui, par sa collerette 8, contre le bourrelet 16 du manchon annulaire élastique 11.

Dans une seconde phase, illustrée sur la figure 3, on provoque l'ouverture de la membrane élastique 4 en bec de canard au moyen du tube intérieur 9 qui, à cet effet, est repoussé en aval du trocart jusqu'à ce que sa collerette 10 vienne coincer le jonc 17 interne du manchon élastique 11 contre la collerette 8 du tube de guidage 7. Dans cette position, comme les lèvres de la membrane élastique 4 reposent exclusivement sur la paroi extérieur du tube intérieur 9, seule la couronne 12 située à l'arrière dudit manchon 11 contribue au maintien de l'instrumentation opératoire le long du trocart. La figure 4 illustre, vue de face, le rôle de la membrane 4 dans cette position.

Dans une dernière phase, décrite en référence à la figure 5, l'instrumentation opératoire est enfoncée le long du trocart de sorte que l'on peut retirer le tube intérieur 9 en butée arrière, dans sa position initiale de la figure 2. Ce déplacement dudit tube 9 amènent les lèvres de la membrane élastique 4 au contact de la paroi extérieure de la pince coelioscopique 13, qui est à cet instant maintenu le long du trocart par le biais desdites lèvres et de la couronne 12 du manchon arrière 11. De cette manière, la pince 13 peut tout à fait être placée à la verticale sans glisser le long du trocart par simple gravité.

## Revendications

**1 -** Trocart notamment destiné à l'introduction et au maintien d'une instrumentation opératoire, du type de pinces (13) ou de systèmes optiques d'éclairage et d'exploration visuelle, dans un pneumopéritoine d'un patient opéré par coelioscopie, ce trocart comportant au moins un tube (1) destiné à être introduit au travers de la peau et du péritoine dudit patient pour procurer un passage coaxial à ladite instrumentation, ledit tube (1) d'introduction étant prolongé à sa partie proximale par un logement (2, 6) dans lequel est disposée une bague annulaire (3), normalement fermée par une membrane élastique (4) faisant étanchéité entre l'extérieur et le pneumopéritoine du patient, caractérisé en ce que ladite membrane élastique (4) présente une forme conique, évasée à sa partie distale, et en ce qu'elle est fendue par son sommet (5) en deux parties égales de manière à former une sorte de "bec de canard".

**2 -** Trocart notamment applicable à la coelioscopie selon la revendication précédente, caractérisé en ce que l'on donne une forme légèrement ovale ou oblongue à la paroi extérieure de la bague annulaire (3) portant la membrane élastique (4) fendue, avec un petit axe parallèle à l'incision pratiquée dans cette dernière, ladite bague annulaire (3) étant disposée à l'intérieur d'un logement (2, 6) ayant la forme d'un cylindre de révolution.

**3 -** Trocart notamment applicable à la coelioscopie selon l'une quelconque des revendications précédentes, caractérisé en ce que la bague annulaire (3) et sa membrane élastique (4) sont réalisées en un matériau élastomère incompressible, normalement utilisé comme ressort industriel.

**4 -** Trocart pour la coelioscopie selon la revendication précédente, caractérisé en ce qu'une échancrure circonférentielle (14) est réalisée à la racine de la membrane élastique (4) conique, cette échancrure (14) facilitant le dégagement des deux parties du "bec de canard" vers l'extérieur lorsqu'un instrument traverse ladite membrane (4).

**5 -** Trocart notamment destiné à l'introduction et au maintien d'une instrumentation opératoire, du type de pinces (13) ou de systèmes optiques d'éclairage et d'exploration visuelle, dans un pneumopéritoine d'un patient opéré par coelioscopie, ce trocart comportant au moins un tube (1) destiné à être introduit au travers de la peau et du péritoine dudit patient pour procurer un passage coaxial à ladite instrumentation, ledit tube (1) d'introduction étant prolongé à sa partie proximale par un logement (2, 6) dans lequel est disposée une bague annulaire (3), normalement fermée par une membrane élastique (4) faisant étanchéité entre l'extérieur et le pneumopéritoine du patient, caractérisé en ce que le logement proximal du tube (1) d'introduction dudit trocart est constitué par une douille cylindrique (2) sur laquelle on vient visser un manchon fileté (6) qui est prolongé, à sa partie proximale, par un tube de guidage (7) servant à faire coulisser un tube intérieur (9) d'ouverture et de fermeture de la membrane élastique (4).

**6 -** Trocart notamment applicable à la coelioscopie selon la revendication précédente, caractérisé en ce que le tube intérieur (9) présente une possibilité de déplacement coaxial limité à l'intérieur du tube de guidage (7).

**7 -** Trocart notamment applicable à la coelioscopie selon l'une quelconque des revendications 5 ou 6, caractérisé en ce que le tube intérieur (9) monté coulissant dans le tube de guidage (7) est pourvu, à son extrémité proximale, d'un manchon annulaire (11) élastique comportant, à l'arrière, une couronne (12) de rayon intérieur adéquat pour former un joint torique servant à centrer et à freiner ou bloquer, par frottement, l'instrumentation pouvant être introduite dans ledit tube intérieur (9).

**8 -** Trocart notamment applicable à la coelioscopie selon la revendication précédente, caractérisé en ce que le tube de guidage (7) et le tube intérieur (9) monté coulissant dans ledit tube de guidage (7) sont chacun pourvus d'une collerette terminale (8, 10), et en ce que le manchon annulaire (11) élastique, disposé à l'arrière dudit tube intérieur (9), est pourvu d'un logement cylindrique (15) intérieur dans lequel sont introduites lesdites collerettes (8, 10), ledit logement (15) étant délimité, à l'avant, par un bourrelet (16) venant serrer de manière élastique la paroi externe dudit tube de guidage (7), tout en servant de butée proximale pour la collerette terminale (8) de ce dernier.

**9 -** Trocart notamment applicable à la coelioscopie selon la revendication précédente, caractérisé en ce que le logement cylindrique (15) intérieur du manchon annulaire (11) comporte deux chambres délimitées par un jonc (17) venant faire étanchéité lorsque les collerettes (10, 8) respectives du tube intérieur (9) et du tube de guidage (7) viennent en butée l'une contre l'autre.
